Europäisches Patentamt
European Patent Office
Office européen des brevets

(19) (11) Publication number: 0 071 433 A1

# EUROPEAN PATENT APPLICATION

(12)

(21) Application number: 82303902.9

(22) Date of filing: 23.07.82

(51) Int. Cl.[3]: A 61 K 37/26
A 61 K 45/06, C 07 D 209/18
C 07 D 295/18
//(A61K37/26, 31/535), (A61K37/26, 31/495), (A61K37/26, 31/405)

(30) Priority: 28.07.81 JP 118172/81
30.06.82 JP 113339/82

(43) Date of publication of application: 09.02.83 Bulletin 83/6

(84) Designated Contracting States: AT BE CH DE FR GB IT LI NL SE

(71) Applicant: KOWA COMPANY, LTD.
6-29, 3-chome Nishiki
Naka-ku Nagoya Aichi-ken(JP)

(72) Inventor: Fujii, Setsuro
1-4-27, Midorigaoka
Toyonaka-shi Osaka-fu(JP)

(72) Inventor: Hattori, Eizou
2-6-21, Tsurumai
Sakado-shi Saitama-ken(JP)

(72) Inventor: Hirata, Mitsuteru
1001, Suneori Tsurugashima-cho
Iruma-gun Saitama-ken(JP)

(72) Inventor: Watanabe, Koichiro
2-17-43, Noguchi-cho
Higashi-Murayama-shi Tokyo(JP)

(72) Inventor: Ishihama, Hiroshi
2-8-8, Suwa-cho
Higashi-Murayama-shi Tokyo(JP)

(74) Representative: Webb, Frederick Ronald et al,
G.F. Redfern & Co. Marlborough Lodge 14 Farncombe Road
Worthing West Sussex BN11 2BT(GB)

(54) **Medicine for the treatment of diabetes.**

(57) A medicine for the treatment of diabetes which comprises insulin and a synthetic phenyl ester type chymotrypsin inhibitor is effectively useful for absorption of insulin through the intestinal canal without the deactivation of insulin in the canal.

EP 0 071 433 A1

"MEDICINE FOR THE TREATMENT OF DIABETES"

This invention relates to a medicine for the treatment of diabetes, and more particularly to such medicine comprising insulin and a synthetic phenyl ester type chymotrypsin inhibitor.

Insulin has been widely used a medicine for treating diabetes in clinical diagnosis. Because of being a polypeptide, however, insulin when administered orally is liable to be deactivated by the action of various proteases in the digestive juice, and hence, does not exhibit its inherent curative effect on diabetes. Accordingly, insulin has heretofore been administered only by intravenous or intramascular injection.

However, the injection of insulin must be performed by experts, and at the same time, diabetes requires treatment by continuous administration of insulin for a prolonged period of time. This induces the drawback that a patient suffers physical and mental pain and feels annoyed about the treatment.

In recent years, research has been directed to the administration of insulin by routes other than injection. There have been disclosed in Japanese Laid-open Application No. 15412/1978 a suppository by which insulin is absorbable through the intestinal canal with use of insulin in combination with a trypsin inhibitor, and in Japanese Laid-open Application No. 107408/1978 a preparation by which insulin is acceleratively absorbed

through the intestinal canal with use of insulin in combination with a surfactant. The known suppository and preparation are not necessarily satisfactory and far from practical.

In order to overcome the above-noted disadvantages of the prior art techniques, the present inventors have made many studies of preparations by which insulin can be absorbed through the intestinal canal by routes other than injection, for example, by an oral or rectal route, thereby producing blood sugar-lowering action. In the studies leading to the present invention, it has been found that as compared to the conventional preparations of insulin combined with natural or synthetic trypsin inhibitors, insulin when administered together with substances which specifically inhibit chymotrypsin is satisfactorily absorbable through the intestinal canal and achieves the desired blood sugar-lowering activities.

Therefore, one object of the present invention is to provide a medicine for the treatment of diabetes which is devoid of the shortcomings of the existing techniques.

A more specific object of the invention is to provide a medicine capable of facilitating the absorption of insulin through the intestinal canal, without insulin being deactivated in the canal.

This and other objects of the invention as hereinafter will become readily apparent can be obtained by

providing a medicine for the treatment of diabetes which comprises insulin and a synthetic phenyl ester type chymotrypsin inhibitor.

A more complete appreciation of the invention and many of the attendant advantages thereof will be readily obtained as the same becomes better understood by reference to the following detailed description when considered in connection with the accompanying drawings, wherein:

FIG. 1 is a diagram showing the relationship between the time course after administration of a medicine embodying the invention and the lowering of blood sugar levels;

FIG. 2 is a diagram showing the effects of insulin administered alone and in combination with other acceptable compounds upon the decreases in blood sugar level; and

FIG. 3 through FIG. 5, inclusive, are diagrams showing the effects of insulin and chymotrypsin inhibitors administered in varied concentrations upon the changes in blood sugar level.

Insulin, which may be used in the present invention, is not particularly limited in respect of its origin. Any species of insulin are applicable so long as these species are absorbed through the intestinal canal and exhibit blood sugar level-lowering activities. Suitable species of insulin which are useful in the

invention include, for example, those originating from cattle, hogs, whales and the like, those derived from microorganisms, cells and the like which have gained insulin-producing action by means of the gene rearrangement operation, and those obtained synthetically.

Synthetic phenyl ester type chymotrypsin inhibitors (hereinafter referred to as "chymotrypsin inhibitors"), which may be employed in the invention, include compounds represented by the formula (I) written below and acid addition salts thereof,

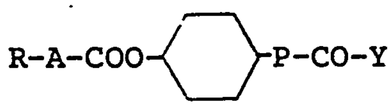 ............ (I)

wherein R is indole which may be substituted with lower alkyl and/or lower alkoxy; naphthyl which may be substituted with lower alkoxy and which may be partially saturated with 2 or 4 hydrogen actoms; phenyl which may be substituted with lower alkyl, alkoxy, alkoxycarbonyloxy, acyl, acyloxy or halogen; cyclohexyl; or indane or furyl;

A is a single bond, alkylene or vinylene;

P is a single bond, alkylene, vinylene or alkylene attached to the benzene ring through an oxygen or nitrogen atom in which said alkylene group may be substituted with amino, alkylamino or benzoyloxy amino, and said nitrogen atom may be substituted with acyl; and

Y is $-N\begin{matrix} R_1 \\ R_2 \end{matrix}$ in which $R_1$ and $R_2$ are same or different, and represents hydrogen, lower alkyl, acyl, aminoalkyl or aminoacyloxyalkyl; -N N-$R_3$ in which $R_3$ represents lower alkyl, cyclo alkyl, benzyl, carbamoyl lower alkyl, morpholinocarbonyl lower alkyl, pyrrolidinocarbonyl lower alkyl, piperidinocarbonyl lower alkyl, piperidino lower alkyl, anilinocarbonyl lower alkyl, alkylaminocarbonyl lower alkyl or alkoxycarbonyl lower alkyl; $-OR_4$ in which $R_4$ represents hydrogen, lower alkyl, aralkyl, succinimido, aminoacyloxyalkyl, aminoalkyloxyalkyl or aminoalkylcarbamoylalkyl; -NH-alkylene -N N-$R_5$ or -O-alkylene-N N-$R_5$ in which $R_5$ represents lower alkyl, morpholine lower alkyl, morpholinocarbonyl lower alkyl, pyrrodinocarbonyl lower alkyl, piperidinocarbonyl lower alkyl or lower alkylaminocarbonyl lower alkyl; or $-OQN\begin{matrix} R_6 \\ R_7 \end{matrix}$ in which Q represents straight-chain or branched-chain alkylene, $R_6$ and $R_7$ each represent lower alkyl, or both jointly form, together with an adjacent nitrogen atom, pyrrolidino, piperidino or morpholino.

The compounds of the formula (I) may be divided into the following three groups.

A) Compounds of the formula (I-a):

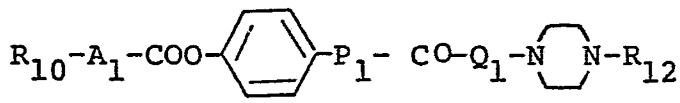 ...(I-a)

wherein $R_{10}$ is indole which may be substituted with lower alkyl or lower alkoxy; naphthyl which may be partially saturated with 2 or 4 hydrogen atoms; phenyl or cyclohexyl which may be substituted with lower alkyl;

$A_1$ is a single bond or alkylene;

$P_1$ is a single bond or vinylene;

$Q_1$ is -O-alkylene or -NH-alkylene when $P_1$ is a single bond; or vinylene when $P_1$ is vinylene; and

$R_{12}$ is lower alkyl, morpholino lower alkyl, morpholinocarbonyl lower alkyl, pyrrolidinocarbonyl lower alkyl, piperidinocarbonyl lower alkyl or lower alkylaminocarbonyl lower alkyl.

B) Compounds of the formula (I-b):

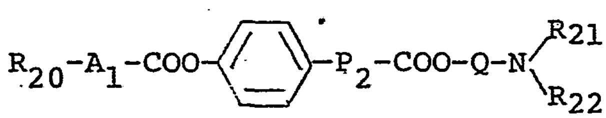 ......(I-b)

wherein $R_{20}$ is indole which may be substituted with lower alkyl and/or alkoxy; or naphthyl or indane which may be partially saturated with 2 or 4 hydrogen atoms;

$P_2$ is a single bond, alkylene or vinylene;

$Q_2$ is straight-chain or branched-chain alkylene;

$R_{21}$ and $R_{22}$ each represent lower alkyl, or both jointly form, together with an adjacent nitrogen atom, pyrrolidino, piperidino or morpholino; and

$A_1$ is as defined above.

C) Compounds of the general formula (I-c):

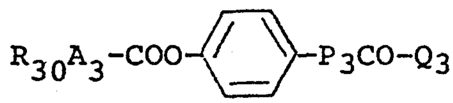 ............. (I-c)

wherein $R_{30}$ is phenyl which may be substituted with 1 to 3 groups of lower alkyl, alkoxy, alkoxycarbonyloxy, acyl, acyloxy or halogen; naphthyl which may be substituted with lower alkoxy and which may be partially saturated with 2 or 4 hydrogen atoms; indole which may be substituted with lower alkyl or alkoxy; indane or furyl;

$A_3$ is a single bond, alkylene or vinylene;

$P_3$ is a single bond, alkylene or alkylene attached to the benzene ring through an oxygen or nitrogen atom in which said alkylene group may be substituted with amino, alkylamino or benzoyloxyamino, and said nitrogen atom may be substituted with acyl; and

$Q_3$ is 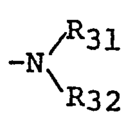 in which $R_{31}$ and $R_{32}$ are same

or different, and represent hydrogen, lower alkyl, acyl, aminoalkyl, or aminoacyloxyalkyl; 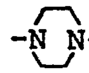$-R_{33}$ in which $R_{33}$ represents lower alkyl, cycloalkyl, benzyl, carbamoyl lower alkyl, morpholinocarbonyl lower alkyl, piperidinocarbonyl lower alkyl, piperidino lower alkyl, anilinocarbonyl lower alkyl, alkylaminocarbonyl lower alkyl or alkoxycarbonyl lower alkyl; or $-OR_{34}$ in which $R_{34}$ represents hydrogen, lower alkyl, aralkyl, succinimido, aminoacyloxyalkyl, aminoalkyloxyalkyl or aminoalkylocarbamoylalkyl.

Some processes for preparing the above-mentioned compounds and chymotrypsin-inhibiting activities of the compounds are disclosed in Japanese Patent Laid-open Applications No. 149240/1980 and No. 158737/1981 and Japanese Patent Applications No. 86569/1981 and No. 110350/1981. A typical example of the processes is illustrated by the following reaction scheme.

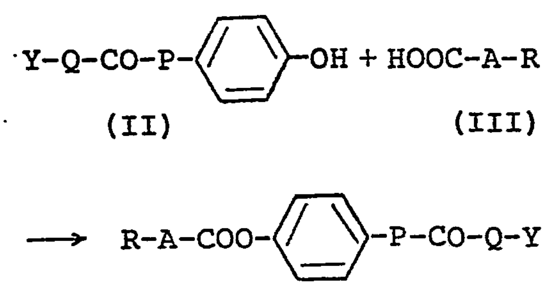

(II) (III)

(I)

wherein R, A, P, Q and Y are the same as defined above.

That is, the chymotrypsin inhibitors which are useful in the invention are easily prepared by esterification of 4-substituted phenols of the formula (II) and carboxylic acids of the formula (III), as is known in the art.

The esterification reaction of the compounds of the formula (II) with the compounds of the formula (III) may be advantageously carried out by reaction of a reactive derivative of the compound of the formula (III), for example, an acid halide, an acid anhydride, a mixed acid anhydride, an active ester or an azide or the like, with the compound of the formula (II), by the active amide process or oxidation-reduction process, by reaction of the compound of the formula (II) with the compound of the formula (III) in the presence of a dehydrating agent such as dicyclohexylcarbodiimide or the like, or by other processes.

The chymotrypsin inhibitors are compounds which are extremely low in toxicity. For example, the acute toxicity ($LD_{50}$ value) of the compounds is about 300 to 400 mg/kg when administered intravenously to mice. Furthermore, such chymotrypsin inhibitors are compounds possessing extremely high chymotrypsin-inhibiting activities, as is clear from their molarity of about $10^{-6}$ to $10^{-8}$ at the 50% inhibition concentration.

Medicines for the treatment of diabetes according to the invention were tested in respect of their pharmacological activities, with the results described hereinafter.

1) Blood sugar level-lowering action of various chymotrypsin inhibitors used in combination with insulin:

Determination method A

Rabbits, each group consisting of 5 rabbits, which had previously been starved overnight and implanted in the duodenum with a catheter, were administered with test medicines through the catheter. Blood was collected from each animal before and 30 minutes after the administration. The blood sugar levels in the collected blood were determined by the glucose oxidase method. Each medicine was prepared by dissolving or suspending 250 units of insulin and 100 mg of one of the chymotrypsin inhibitors given below in 20 mℓ of water, and the resulting solution or suspension was administered in a dose of 2 mℓ/kg.

The blood sugar level-decreasing ratios were calculated by the following equation on the basis of the blood sugar levels determined above.

The results obtained are shown in Table 1.

$$\text{Blood sugar level-decreasing ratio (\%)} = \frac{\begin{pmatrix}\text{Blood sugar}\\ \text{level before}\\ \text{administration}\end{pmatrix} - \begin{pmatrix}\text{Blood sugar level}\\ \text{30 minutes after}\\ \text{administration}\end{pmatrix}}{\text{Blood sugar level before administration}} \times 100$$

Table 1

| Chymotrypsin inhibitor (Compound No.) | Blood sugar level-decreasing ratio (%) |
|---|---|
| 1 | 20 |
| 2 | 25 |
| 3 | 22 |
| 4 | 29 |
| 5 | 36 |
| 6 | 44 |
| 7 | 37 |
| 8 | 32 |
| 9 | 35 |
| 10 | 51 |
| 11 | 48 |
| 12 | 26 |
| 13 | 29 |
| 14 | 37 |
| 15 | 32 |
| 16 | 26 |
| 17 | 40 |
| 18 | 35 |
| 19 | 32 |
| Blank | 0 |

The compounds used as chymotrypsin inhibitors in this test were as follows:

1. 4-[[2-[4-(Morpholinocarbonylmethyl)piperadino]ethyl]oxycarbonyl]phenyl cyclohexylcarboxylate dihydrochloride
2. 4-[[2-[4-(Morpholinocarbonylmethyl)piperadino]ethyl]oxycarbonyl]phenyl 1,2,3,4-tetrahydro-1-naphthoate dihydrochloride
3. 4-[[2-[4-(Morpholinocarbonylmethyl)piperadino]ethyl]oxycarbonyl]phenyl 4-methylbenzoate

dihydrochloride

4. 4-[[2-[4-(Morpholinocarbonylmethyl)piperadino]ethyl]oxycarbonyl]phenylnaphthyl-1-acetate dihydrochloride

5. 4-[[2-[4-(Morpholinocarbonylmethyl)piperadino]ethyl]oxycarbonyl]phenyl 5-methoxy-2-methylindole-3-acetate dihydrochloride

6. 4-[(2-Diethylamino-1-methylethyl)oxycarbonyl]-phenyl 5-methoxy-2-methylindole-3-acetate

7. 4-[2-[2-Diethylamino-1-methylethyl)oxycarbonyl]ethenyl]phenyl 5-methoxy-2-methylindole-3-acetate

8. 4-[(2-Dimethylaminoethyl)carbamoyl]phenyl 5-methoxyindole-3-acetate

9. 4-[2-[(2-Piperidinoethyl)oxycarbonyl]ethenyl]-phenyl 5-methoxy-2-methylindole-3-acetate

10. 4-[2-[(2-Dimethylamino-1-methylethyl)oxycarbonyl]ethenyl]phenyl 5-methoxy-2-methylindole-3-acetate

11. 4-[(2-Piperidinoethyl)oxycarbonyl]phenyl 5-methoxy-2-methylindole-3-acetate

12. 4-[[2-(1,5-Diamino-n-pentylcarbonyloxy)-ethyl]oxycarbonylmethyl]phenyl 5-methoxy-2-methylindole-3-acetate dihydrochloride

13. 4-[[2-(1,5-Diamino-n-pentylcarbonyloxy)-ethyl]carbamoylmethyl]phenyl 5-methoxy-2-methylindole-3-acetate dihydrochloride

14. 4-[[2-(1,5-Diamino-n-pentylcarbonyloxy)-ethyl]oxycarbonylmethyl]phenyl 1,2,3,4-

tetrahydro-1-naphthoate dihydrochloride

15. 4-[[2-(1,5-Diamino-n-pentylcarbonyloxy)ethyl]-carbamoylmethyl]phenyl 1,2,3,4-tetrahydro-1-naphthoate dihydrochloride
16. 4-[(2-Dimethylaminoethyl)carbamoyl]phenyl 5,6,7,8-tetrahydro-1-naphthoate hydrochloride
17. 4-[(2-dimethylaminoethyl)carbamoyl]phenyl 5-methoxy-2-methylindol-3-acetate maleate
18. 4-[(2-Dimethylaminoethyl)carbamoyl]phenyl 1,2,3,4-tetrahydro-1-naphthoate hydrochloride
19. 1-Methyl-4-[4-(1,2,3,4-tetrahydro-1-naphthoyloxy)benzoyl]piperazine hydrochloride

Determination method B

Male rats, each group consisting of 5 rats weighing 180 to 210 g, were subjected to abdominal operation under anesthetization with urethane-chloralose and administered with test medicine liquids through an injection needle into the duodenum 2 cm apart from the pyloric region. Before and 1 hour after the administration, blood was collected from the lower venous trunk of each rat. The blood sugar levels of the collected blood were determined by the glucose oxidase method. Each medicine liquid was prepared by dissolving or suspending 250 units of insulin and 20 mg and 40 mg of one of the chymotrypsin inhibitors given below in 2 mℓ of water.

The blood sugar level-decreasing ratios were calculated by the following equation on the basis of the blood sugar levels predetermined above.

The results obtained are shown in Table 2.

$$\text{Blood sugar decreasing ratio (\%)} = \frac{\left[\begin{array}{l}\text{Blood sugar}\\ \text{level before}\\ \text{administration}\end{array}\right] - \left[\begin{array}{l}\text{Blood sugar level}\\ \text{1 hour after}\\ \text{administration}\end{array}\right]}{\text{Blood sugar level before administration}} \times 100$$

Table 2

| Chymotrypsin inhibitor (Compound No.) | Blood sugar level-decreasing ratio (%) | |
|---|---|---|
| | Dose of 20 mg/kg | Dose of 40 mg/kg |
| 1 | 16 | 52 |
| 2 | 50 | 64 |
| 3 | 41 | 53 |

The compounds used as chymotrypsin inhibitors in this test were as follows:

1. 1-Isopropylaminocarbonylmethyl)-4-[4-(1,2,3,4-tetrahydro-1-naphthoyloxy)benzoyl]piperazine methanesulfonate
2. 1-Isopropyl-4-[4-(1,2,3,4-tetrahydro-1-naphthoyloxy)benzoyl]piperazine methanesulfonate
3. 1-Ethyl-4-[4-(1,2,3,4-tetrahydro-1-naphthoyloxy)benzoyl]piperazine methanesulfonate

Determination method C

Beagle dogs, each group consisting of 6 dogs, which had been starved overnight, were orally administered with test medicines, and blood was collected from the animals before administration and 0.5, 1, 1.5, 2, 2.5, 3, 4 and 5 hours after the administration. The blood sugar levels of the collected blood were determined by the same method as adopted in determination

method A. Each medicine was prepared by blending 25 units/kg of insulin with 10 mg/kg of a chymotrypsin inhibitor, and the resulting blend was encapsulated with a gelatin hard capsule coated with a coat soluble in the intestines.

The results obtained are shown in FIG. 1 wherein the value is an average value of 6 dogs of one group.

The compounds used as chymotrypsin inhibitors in this test were as follows:

1. 1-(Isopropylaminocarbonylmethyl)-4-[4-(1,2,3,4-tetrahydro-1-naphthoyloxy)benzoyl]piperazine methanesulfonate
2. 1-(Piperidinocarbonylmethyl)-4-[4-(1,2,3,4-tetrahydro-1-naphthoyloxy)benzoyl]piperazine methanesulfonate
3. 1-Isopropyl-4-[4-(1,2,3,4-tetrahydro-1-naphthoyloxy)benzoyl]piperazine methanesulfonate
4. 1-(Dimethylaminocarbonylmethyl)-4-[4-(1,2,3,4-tetrahydro-1-naphthoyloxy)benzoyl]piperazine methanesulfonate

2) Comparative test between chymotrypsin inhibitors and trypsin inhibitors:

Rabbits which had been treated in the same manner as adopted in determination method A of item 1) above were administered with test medicines. Blood of the rabbits was collected before the administration and 30 minutes and 90 minutes after the administration, and the blood sugar levels of the collected blood were determined. The medicines were administered in a dose

 

of 25 units/kg of insulin in combination of 20 mg/kg of a chymotrypsin inhibitor or 10, 20 and 40 mg/kg of a trypsin inhibitor in the same manner as employed in determination method A of item 1) above.

The results obtained are shown in FIG. 2.

The chymotrypsin inhibitors used were 4-[[2-[4-[2-(morpholino)ethyl]piperazino]ethyl]oxycarbonyl]-phenyl 5-methoxy-2-methylindole-3-acetate trihydrochloride (1) and 1-ethyl-4-[4-(1,2,3,4-tetrahydro-1-naphthoyloxy)benzoyl]piperazine methanesulfonate (2), and the trypsin inhibitors used were leupeptine and mesyl gabexate.

3) Reaction test by dosage:

Determination method A

Overnight-starved beagle dogs, each group consisting of 3 dogs, were orally administered with test medicines, and blood was collected from each of the animals before the administration and 0.5, 1, 1.5, 2, 3, 4 and 5 hours after the administration. The blood sugar levels of the collected blood were determined by the same manner as used in item 1) above. Each medicine was prepared by blending insulin and a chymotrypsin inhibitor in the ratio given below, and the resulting blend was encapsulated in a gelatin hard capsule coated with a coat soluble in the intestines.

(1) Chemicals for reaction of chymotrypsin inhibitor by dosage: prepared by blending insulin with a chymotrypsin inhibitor in a ratio of 25 units/kg of insulin to 10 mg/kg,

20 mg/kg and 40 mg/kg of chymotrypsin inhibitor

(2) Chemicals for reaction of insulin by dosage: prepared by blending a chymotrypsin inhibitor with insulin in a ratio of 20 mg/kg of chymotrypsin inhibitor to 12.5 units/kg, 25 units/kg and 50 units/kg of insulin

The compound used as a chymotrypsin inhibitor in this test was the compound (1) identified in item 2) above.

The results obtained are shown in FIGS. 3 and 4.

Determination method B

Using the same procedure as in determination method B of item 1) above and beagle dogs, a reaction of a chymotrypsin inhibitor by dosage was investigated by determining the blood sugar levels. Each test medicine was prepared by blending insulin with a chymotrypsin inhibitor in a ratio of 25 units/kg of insulin to 5 mg/kg and 10 mg/kg of chymotrypsin inhibitor and encapsulating the blend in a gelatin hard capsule coated with a coat in the same manner as in determination method B in of item 1). As the chymotrypsin inhibitor, use was made of 1-isopropyl-4-[4-(1,2,3,4-tetrahydro-1-naphthoyloxy)benzoyl]piperazine methanesulfonate.

The results obtained are shown in FIG. 5.

As is clear from the foregoing results, it has been confirmed that insulin when administered together with chymotrypsin inhibitors is absorbed in the intestinal canal and exhibits substantially excellent sugar

level-lowering activities. Such activities accruing from the medicines of the invention are about five to ten times as high as those of the case where insulin is used in combination with known trypsin inhibitors.

For the practice of the invention, it is desired that the chymotrypsin inhibitor be used in combination with insulin in a ratio of 0.001 to 50 mg of the former to one unit of the latter. Any modes of administration wherein both insulin and the chymotrypsin inhibitor can coexist in the intestinal canal may be suitably utilized for the invention. A more preferable mode is to use insulin together with the chymotrypsin inhibitor, to use both components separately in the form of a preparation for an oral route such as a tablet or a capsule soluble in the intestines or the like, or to use both components in the form of a suppository.

Suitable doses of the medicines according to the invention range from 20 to 5,000 units in terms of insulin for an adult per one time, depending upon the blood sugar level of a patient, the mode of administration and other conditions. Particularly preferable is the range of 50 to 2,000 units.

The above disclosure generally describes the present invention. A more complete understanding will be obtained by the following specific examples and reference examples which are provided for purposes of illustration only and are not construed as limiting to the invention.

Example 1

To a mixture of 400 units of insulin and 320 mg of a chymotrypsin inhibitor were added suitable amounts of crystalline cellulose, carboxymethyl cellulose calcium and talc. The resulting mixture was encapsulated in a gelatin capsule and further coated with an enteric coat, whereby a capsule preparation was obtained.

Example 2

To a mixture of 300 units of insulin and 150 mg of a chymotrypsin inhibitor were added suitable amounts of crystalline cellulose, carboxymethyl cellulose calcium, hydroxypropyl cellulose and magnesium stearate. The resulting mixture was formed into a tablet which was then coated with an enteric coat, whereby a tablet preparation was obtained.

Example 3

To a mixture of 625 units of insulin and 500 mg of a chymotrypsin inhibitor were added suitable amounts of crystalline cellulose, carboxymethyl cellulose calcium and hydroxypropyl cellulose. The resulting mixture was granulated, and the granules were coated with an enteric coat, whereby a granular preparation was obtained.

Example 4

A mixture of 50 units of insulin and 50 mg of a chymotrypsin inhibitor was blended with a suitable amount of Witepsol H 19 to prepare a suppository.

Reference Example 1

4-[[2-[4-[2-(Morpholino)ethyl]piperazino]ethyl]-oxycarbonyl]phenyl 5-methoxy-2-methylindole-3-acetate:

(1) To a solution of 623 g of morpholine ethanol in 2 ℓ of benzene, kept at an internal temperature of less than 35°C, was added dropwise with stirring and ice-cooling a solution of 735 g of thionyl chloride in 500 mℓ of benzene. After completion of the dropwise addition, the reaction liquid was refluxed with stirring for 4 hours. After cooling, the deposited crystals were separated and collected to obtain 872.4 g (yield: 98.7%) of chloroethylmorpholine hydrochloride, m.p. 180 to 182°C.

A mixture of 223 g of the thus obtained chloroethylmorpholine hydrochloride, 130 g of piperazine ethanol, 336 mℓ of triethylamine and 36 g of sodium iodide was susepnded in 1 ℓ of toluene, followed by refluxing with stirring for 2 hours. After being cooled, the reaction liquid was filtered to remove insolubles, and the solvent was removed by distillation to obtain 209 g of an oily product.

To a solution of the thus obtained oily product and 161 mℓ of triethylamine in 1 ℓ of ethyl acetate was added dropwise with stirring and ice-cooling 300 mℓ of an ethyl acetate solution of 262 g of an acid chloride prepared, according to the usual way, from 240 g of p-ethoxycarbonyloxybenzoic acid, followed by stirring at room temperature for 3 hours. The reaction liquid

was filtered to remove precipitates and acidified with 2N-hydrochloric acid. The hydrochloric acid layer separated and collected was washed with ethyl acetate, neutralized with an aqueous sodium bicarbonate solution and then extracted with chloroform. The chloroform layer separated and collected was washed with water, dried and then concentrated. The deposited crystals were recrystallized from ethyl acetate-n-hexane to obtain 329 g (yield: 77.4%) of 2-[4-[2-(morpholino)-ethyl]piperazino]ethyl 4-ethoxycarbonyloxybenzoate as colorless crystals, m.p. 85 to 87°C.

Subsequently, the thus obtained 4-ethoxycarbonyloxy benzoate was dissolved in 1.8 ℓ of ehtyl acetate, and 64.6 mℓ of pyrrolidine was added to the solution. The resulting mixture was stirred at room temperature for 30 minutes and then allowed to stand overnight, and the resulting precipitates were collected by filtration. The precipitates were recrystallized from ethanol obtain 204 g (yield: 72.6%) of 2-[4-[2-(morpholino)ethyl]piperazino]ethyl 4-hydroxybenzoate as colorless crystals, m.p. 176 to 178°C.

(2) A mixture of 204 g of the 4-hydroxybenzoate obtained in item(1), 148 g of 5-methoxy-2-methyl-3-indoleacetic acid, 138 g of dicyclohexylcarbodiimide and 8.5 g of 4-dimethylaminopyridine was dissolved in 3 ℓ of acetonitrile and then stirred at room temperature for 4 hours. Followed by removal of deposited insolubles by filtration from the reaction liquid, the solvent was removed by distillation. The reaction liquid thus

treated was dissolved in 450 mℓ of 2N-hydrochloric acid and washed with ethyl acetate. The hydrochloric acid layer was neutralized with sodium bicarbonate and extracted with chloroform. The chloroform layer was washed with water and dried, and the solvent was removed by distillation to obtain a dark redish oily product. This oily product was dissolved in 3.5 ℓ of a mixed solvent of acetonitrile and ethanol(4:1) and incorporated with a calculated amount of a hydrochloric acid-dioxane solution, and the resulting mixture was allowed to stand overnight under cool conditions. The deposited crystals were collected by filtration and dried to obtain 294 g (yield: 77.7%) of 4-[[2-[4-[2-(morpholino)ethyl]-piperazino]ethyl]oxycarbonyl]phenyl 5-methoxy-2-methylindole-3-acetate trihydrochloride as pale yellow crystals, m.p. 231 to 233°C.

Reference Example 2

4-[[2-[4-(Morpholinocarbonylmethyl)piperazino]-ethyl]carbamoyl]phenyl 5-methoxy-2-methylindole-3-acetate:

(1) A solution of 25.4 g of phenyl 4-(benzyloxy)-benzoate and 21.6 g of N-(2-aminoethyl)piperazine in chloroform was heated for 5 hours under reflux conditions. After completion of the reaction, a residue obtained by concentrating the reaction liquid under reduced pressure was dissolved in ethyl acetate and extracted with 105 mℓ of 4N-hydrochloric acid. The hydrochloric acid layer collected by separation was neutralized with cooling by addition of 17 g of sodium

hydroxide. The neutralized layer was extracted with chloroform, washed with saturated saline, dried and distilled to remove the solvent to deposit crystals. The crystals are recrystallized from a mixed solvent of ethyl acetate and ether to obtain 8.5 g (yield: 30.0%) of N-[2-piperazino)ethyl] 4-(benzyloxy)benzamide as colorless crystals, m.p. 116 to 118°C.

To 80 mℓ of an ethanol solution of 8.0 g of the thus obtained benzamide, 3.45 g of potassium carbonate and 0.37 g of sodium iodide was added 7.72 g of N-(chloroacetyl)morpholine, and the resulting mixture was heated for 5 hours under reflux conditions. After completion of the reaction, insolubles were removed by filtration from the reaction liquid, and a residue obtained by removal of the solvent by distillation was dissolved in 90 mℓ of 1N-hydrochloric acid. The resulting hydrochloric acid solution was washed with ethyl acetate, neutralized with sodium hydrogencarbonate and then extracted with 500 mℓ of ethyl acetate. The extract was dried, concentrated under reduced pressure and purified by silica gel chromatography using a mixed solvent of chloroform and methanol (10:1) to obtain 5.3 g (yield: 48.1%) of N-[2-[4-(morpholinocarbonylmethyl)piperazino]-ethyl] 4-(benzyloxy)benzamide as colorless crystals, m.p. 149 to 151°C.

To a solution of 5.2 g of the thus obtained benzamide in 80 mℓ of methanol was added 1 g of 10% palladium-carbon, and the resulting mixture was subjected to catalytic reduction at 40°C for 3 hours. Then,

the reduction product was purified by the conventional method to obtain 4.18 g (quantitative) of N-[2-[4-morpholinocarbonylmethyl)piperazino]ethyl] 4-hydroxybenzamide as an oily product.

Elementary analysis: for $C_{19}H_{28}N_4O_4$

| | | C | H | N |
|---|---|---|---|---|
| Calculated | (%) | 60.62 | 7.50 | 14.88 |
| Found | (%) | 60.34 | 7.41 | 14.70 |

(2) To 30 mℓ of an acetonitrile solution of 4.18 g of the benzamide obtained in item (1), 3.65 g of 5-methoxy-2-methylindole-3-acetic acid and 0.2 g of 4-dimethylamino pyridine was added 3.43 g of dicyclohexylcarbodiimide, and the resulting mixture was stirred at room temperature for 2 hours. Followed by removal of insolubles by filtration from the reaction liquid, a residue obtained by concentration under reduced pressure of the solvent was dissolved in ethyl acetate and extracted with 20 mℓ of 2N-hydrochloric acid. The hydrochloric acid layer collected was neutralized with sodium carbonate and extracted with ethyl acetate. The extract was washed with water and dried, and the solvent was removed by distillation to obtain an oily product. To a solution of the thus obtained oily product in acetonitrile was added 1.73 g of maleic acid, whereby 5.2 g (yield: 86.3%) of 4[[2-[4-(morpholinocarbonylmethyl)-piperazino]ethyl]-carbamoyl]phenyl 5-methoxy-2-methylindole-3-acetate dimaleate was obtained as colorless crystals, m.p. 151 to 153.5°C.

Reference Example 3

1-(Pyrrolidinocarbonylmethyl)-4-[4-(1,2,3,4-tetrahydro-1-naphthoyloxy)cinnamoyl]piperazine:

(1) To a solution of 17.7 g of 1-(pyrrolodinocarbonylmethyl)piperazine and 8.4 mℓ of triethylamine was added with ice-cooling 15.2 g of 4-(ethoxycarbonyloxy)cinnamoyl chloride, and the resulting mixture was stirred overnight at room temperature. The reaction liquid was concentrated under reduced pressure, and the concentrate was added with ethyl acetate and filtered to remove insolubles. Thereafter, the filtrate was extracted with 140 mℓ of 1N-hydrochloric acid. The extract was washed with ethyl acetate, neutralized with sodium hydrogencarbonate and then extracted with chloroform. The chloroform layer was washed with water and then with saturated saline and dried. The crystals obtained by removal of the solvent by distillation were recrystallized from ethyl acetate to obtain 13.4 g (yield: 54.0%) of 1-[4-(ethoxycarbonyloxy)cinnamoyl]-4-(pyrrolidinocarbonylmethyl)piperazine. The piperazine thus obtained was dissolved in 150 mℓ of chloroform, and the solution was charged with 2.3 g of pyrrolidine and stirred at room temperature for 1.5 hours. After completion of the reaction, a residue obtained by removal of the solvent by distillation from the reaction liquid was purified by silica gel chromatography using a mixed solvent of chloroform and methanol (10:1), whereby 11.1 g (quantitative) of 1-(4-hydroxycinnamoyl)-4-(pyrrolidinocarbonylmethyl)-piperazine was obtained as a colorless oily product.

Elementary analysis: for $C_{19}H_{25}N_3O_3$

| | C | H | N |
|---|---|---|---|
| Calculated (%): | 66.45 | 7.34 | 12.24 |
| Found (%): | 66.24 | 7.28 | 12.09 |

(2) Using 1-(4-hydroxycinnamoyl)-4-(pyrrolidinocarbonylmethyl)piperazine obtained in item (1) and 1,2,3,4-tetrahydro-1-naphthoyl chloride, the same procedure as in item (1) of Reference Example 1 was repeated to obtain 1-(pyrrolidinocarbonylmethyl)-4-[4-(1,2,3,4-tetrahydro-1-naphthoyloxy)cinnamoyl]piperazine hydrochloride as crystals, m.p. 237.5 to 240.5°C (yield: 49.5%).

Reference Example 4

Ethyl N-benzyloxycarbonyl-O-(β-indoleacetyl)-L-tyrosinate:

To 50 mℓ of a dimethylformamide solution of 17.2 g (50 mmols) of ethyl N-benzyloxycarbonyl-L-tyrosinate and 19.26 g (110 mmols) of β-indoleacetic acid was added 22.7 g (110 mmols) of dicyclohexylcarbodiimide, and the resulting mixture was stirred overnight at room temperature. The reaction liquid charged with 400 mℓ of ethyl acetate was distilled to remove insolubles and washed with a saturated sodium bicarbonate solution and saturated saline. The reaction liquid thus treated was dried over anhydrous sodium sulfate, concentrated under reduced pressure and purified by silica gel chromatography (elution solvent: chloroform-methanol, 50:1) to obtain 13.3 g (yield: 53.1%) of colorless crystals,

m.p. 101.5 to 103°C.

Elementary analysis: for $C_{29}H_{28}N_2O_6$

| | C | H | N |
|---|---|---|---|
| Calculated (%): | 69.59 | 5.64 | 5.60 |
| Found (%): | 69.45 | 5.63 | 5.41 |

Reference Example 5

Ethyl N-benzyloxycarbonyl-O-(1-naphthylacetyl)-L-tyrosinate:

To 12 mℓ of an ethyl acetate solution of 1.72 g (5 mmols) and 1.4 mℓ (10 mmols) of triethylamine was added dropwise with ice-cooling 2.05 g (10 mmols) of 1-naphtylacetyl chloride. The mixture was stirred with ice-cooling, and the reaction liquid was distilled to remove insolubles and concentrated under reduced pressure. The residue was purified by silica gel chromatography (elution solvent: chloroform) to obtain 1.44 g (yield: 56.2%) of colorless crystals, m.p. 86 to 87°C.

Elementary analysis: for $C_{31}H_{29}NO_6$

| | C | H | N |
|---|---|---|---|
| Calculated (%): | 72.78 | 5.71 | 2.74 |
| Found (%): | 72.99 | 5.71 | 2.51 |

Reference Example 6

N-(2-Dimethylaminoethyl)-N-(benzoyl)-p-(benzoyloxy)benzamide hydrochloride:

To 25 mℓ of a dimethylformamide solution of 4.07 g (19.5 mmols) and 5.6 mℓ (40 mmols) of triethylamine was added dropwise with ice-cooling 4.67 mℓ (40 mmols) of benzoyl chloride. After being stirred at

room temperature for 2 hours, the reaction liquid was distilled to remove insolubles, and the filtrate charged with 50 mℓ of 0.5N-hydrochloric acid was washed with ethyl acetate. The aqueous layer was neutralized with a saturated sodium bicarbonate solution and extracted with ethyl acetate. The extract was washed with saturated saline, dried over anhydrous sodium sulfate and then concentrated under reduced pressure to obtain crude crystals.

The crude crystals thus obtained were purified by silica gel chromatography (elution solvent: chloroform-methanol, 5:1) to obtain 2.70 g of crystals. The crystals were dissolved in 10 mℓ of chloroform, added with a solution of a dioxane solution containing an equimolar amount of hydrogen chloride and then added with ether, whereby 2.88 g (yield: 35.5%) of colorless crystals, m.p. 185 to 186°C was obtained.

Elementary analysis: for $C_{25}H_{24}N_2O_4 \cdot HC\ell$

| | C | H | N |
|---|---|---|---|
| Calculated (%): | 66.30 | 5.56 | 6.18 |
| Found (%): | 66.24 | 5.42 | 6.08 |

Reference Example 7

4-[(2-Dimethylamino-1-methylethyl)oxycarbonyl]-phenyl 5-methoxy-2-methylindole-3-acetate:

A mixture of 59 g of 5-methoxy-2-methyl-3-indoleacetic acid, 50 g of 2-dimethylamino-1-methylethyl-4-hydroxylbenzoate, 56 g of dicyclohexylcarbodiimide and 3.3 g of 4-dimethylaminopyridine was dissolved in

a mixed solvent containing 500 mℓ of acetonitrile and 500 mℓ of ethyl acetate, and the resulting solution was stirred at room temperature for 4 hours. After removal of insolubles by filtration from the reaction liquid, a residue obtained by removal of the solvent by distillation was dissolved in ethyl acetate and acidified with 1N-hydrochloric acid. The hydrochloric acid layer was neutralized with sodium bicarbonate and extracted with chloroform. The extract was washed with water, dried and then distilled to remove the solvent to obtain a yellow oily product. To a solution of the yellow oily product in 500 mℓ of ethanol was added 20 g of oxalic acid, and the resulting mixture was stirred at room temperature for 1 hour. The deposited crystals were recrystallized from ethyl acetate-ethanol to obtain 45 g (yield: 39.1%) of 4-[(2-dimethylamino-1-methylethyl) oxycarbonyl]phenyl 5-methoxy-2-methylindole-3-acetate oxalate as pale yellow crystals, m.p. 106 to 108°C.

Reference Example 8

4-[[2-(Morpholino)ethyl]oxycarbonyl]phenyl 5-methoxy-2-methylindole-3-acetate:

To 30 mℓ of an acetonitrile solution of 3.68 g of 5-methoxy-2-methyl-3-indoleacetic acid, 2.81 g of 2-(morpholino)ethyl 4-hydroxybenzoate and 0.21 g of 4-dimethylaminopyridine was added 3.47 g of dicyclohexylcarbodiimide, and the resulting mixture was stirred overnight at room temperature. After removing insolubles by distillation from the reaction liquid, a residue obtained by removal of the solvent by distillation was

dissolved in ethyl acetate, and the resulting solution was charged with 10 mℓ of 2N-hydrochloric acid. The hydrochloric acid layer collected was neutralized with sodium bicarbonate and extracted with ethyl acetate. After being washed with water and dried, the extract was removed by distillation to obtain an oily product. The oily product was dissolved in 40 mℓ of acetonitrile and charged with a calculated amount of a hydrochloric acid-dioxane solution, whereby 4.34 g (yield: 67.2%) of 4-[[2-(morpholino)ethyl]oxycarbonyl]phenyl 5-methoxy-2-methylindole-3-acetate hydrochloride was obtained as colorless crystals, m.p. 157 to 160°C.

Reference Example 9

1-Methyl-4-[4-(7-methoxy-1,2,3,4,-terahydro-1-naphthoyloxy)benzoyl]piperazine:

To 100 mℓ of an acetonitrile solution of 4.4 g of 1-methyl-4-(4-hydroxybenzoyl)piperazine and 4.94 g of 7-methoxy-1,2,3,4-tetrahydro-1-naphthyl carboxylic acid was added 4.94 g of dicyclohexylcarbodiimide, and the resulting mixture was stirred overnight. After removing insolubles by filtration from the reaction liquid, the filtrate was concentrated under reduced pressure, and the concentrate charged with 50 mℓ of 0.5N-hydrochloric acid was washed with ethyl acetate. The aqueous layer was neutralized with a sodium bicarbonate solution and extracted with ethyl acetate. After being washed with water and dried, the extract was distilled to remove the solvent to obtain a crude oily product. The crude oily product was purified by silica gel chromatography

(elution solvent: chloroform-methanol, 30:1) to obtain 7.57 g of an oily product.

Using the usual method, the oily product thus obtained was converted into a corresponding methanesulfonate to obtain 6.2 g (yield: 61.6%) of pale yellow needles, m.p. 150 to 151°C.

Reference Example 10

1-Ethoxycarbonylmethyl-4-[4-(1,2,3,4-tetrahydro-1-naphthoyloxy)benzoyl]piperazine:

To 50 mℓ of an ethyl acetate solution of 5.52 g of 1-ethoxycarbonylmethyl-4-(4-hydroxybenzoyl)piperazine and 2.83 mℓ of triethylamine was added dropwise with ice-cooling 10 mℓ of an ethyl acetate solution of 3.95 g of 1,2,3,4-tetrahydro-1-naphthoylchloride. The resulting mixture was stirred at room temperature for 4 hours, neutralized with sodium bicarbonate and then extracted with chloroform. After being washed with water and dried, the extract charged with 30 mℓ of ethanol was converted by the usual method into a corresponding methanesulfonate, whereby 8.0 g (yield: 87.1%) of 1-ethoxycarbonylmethyl-4-[4-(1,2,3,4-trtrahydro-1-naphthoyloxy)benzoyl]piperazine methanesulfonate was obtained as colorless crystals, m.p. 149 to 151.5°C.

Elementary analysis: for $C_{26}H_{30}N_2O_5 \cdot CH_3SO_3H$

| | C | H | N |
|---|---|---|---|
| Calculated (%): | 59.33 | 6.27 | 5.12 |
| Found (%): | 59.35 | 6.29 | 5.06 |

Reference Examples 11 to 24

Using any one procedure of Reference Examples 1 to 10, the following compounds were obtained.

11. 1-Cyclohexyl-4-[4-(1,2,3,4-tetrahydro-1-naphthoyloxy)benzoyl]piperazine methanesulfonate

    yield : 86.2%

    appearance : colorless needles

    m.p. : 205 to 207°C

12. 1-Isopropyl-4-[4-(1,2,3,4-tetrahydro-1-naphthoyloxy)benzoyl]piperazine methanesulfonate

    yield : 89.7%

    appearance : colorless needles

    m.p. : 180 to 182°C

13. 1-Ethyl-4-[4-(1,2,3,4-tetrahydro-1-naphthoyloxy)benzoyl]piperazine methanesulfonate

    yield : 69.2%

    appearance : colorless needles

    m.p. : 140 to 143°C

14. 1-Methyl-4-[4-(4-methoxy-1,2,3,4-tetrahydro-1-naphthoyloxy)benzoyl]piperazine

    yield : 56.8%

    appearance : colorless oily product

    m.p. : —

15. 1-Benzyl-4-[4-(1,2,3,4-tetrahydro-1-naphthoyloxy)benzoyl]piperazine methanesulfonate

yield : 77.4%

appearance : colorless needles

m.p. : 188 to 190°C

16. 1-Methyl-4-[4-(6-methoxy-1,2,3,4-tetrahydro-1-naphthoyloxy)benzoyl]piperazine

yield : 42.2%

appearance : oily product

m.p. : —

17. 1-Isopropylaminocarbonylmethyl-4-[4-(7-methoxy-1,2,3,4-tetrahydro-1-naphthoyloxy)-benzoyl]piperazine

yield : 54.1%

appearance : colorless oily product

m.p. : —

18. 1-Isopropylaminocarbonylmethyl-4-[4-(1,2,3,4-tetrahydro-1-naphthoyloxy)benzoyl]piperazine methanesulfonate

yield : 70.0%

appearance : colorless needles

m.p. : 186 to 188°C

19. 1-Pyperizinocarbonylmethyl-4-[4-(1,2,3,4-tetrahydro-1-naphthoyloxy)benzoyl]piperazine methanesulfonate

yield : 75.6%

appearance : colorless needles

m.p. : 199 to 203.5°C

20. 1-Piperizinoethyl-4-[4-(1,2,3,4-tetrahydro-1-naphthoyloxy)benzoyl]piperazine dihydrochloride

yield : 63.1%
appearance : pale yellow needles
m.p. : > 270°C

21. 1-Morpholinocarbonylmethyl-4-[4-(1,2,3,4-tetrahydro-1-naphthoyloxy)benzoyl]piperazine methanesulfonate

yield : 75.6%
appearance : colorless needles
m.p. : 211 to 215°C

22. 1-Dimethylaminocarbonylmethyl-4-[4-1,2,3,4-tetrahydro-1-naphthoyloxy)benzoyl]piperazine methanesulfonate

yield : 74.0%
appearance : colorless needles
m.p. : 182 to 185°C

23. 1-Isopropylaminocarbonylethyl-4-[4-(1,2,3,4-tetrahydro-1-naphthoyloxy)benzoyl]piperazine

yield : 82.0%
appearance : oily product
m.p. : —

24. 1-Carbamoylmethyl-4-[4-(1,2,3,4-tetrahydro-1-naphthoyloxy)benzoyl]piperazine methanesulfonate

yield : 82.4%

appearance : colorless needles

m.p. : 207 to 212°C

This invention now being fully described, it is apparent to those versed in the art that many changes and modifications can be made to the invention without departing the spirit or scope of the invention set forth herein.

CLAIMS:

1. A medicine for the treatment of diabetes, comprising insulin and a synthetic phenyl ester type chymotrypsin inhibitor.

2. The medicine according to claim 1, characterised in that said synthetic phenyl ester type chymotrypsin inhibitor is a compound represented by the following formula or an acid addition salt thereof,

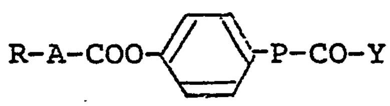

wherein R is indole which may be substituted with lower alkyl and/or lower alkoxy; naphthyl which may be substituted with lower alkoxy and which may be partially saturated with 2 or 4 hydrogen atoms; phenyl which may be substituted with lower alkyl, alkoxy, alkoxycarbonyloxy, acyl, acyloxy or halogen; cyclohexyl; or indane or furyl;

A is a single bond, alkylene or vinylene;

P is a single bond, alkylene, vinylene or alkylene attached to the benzene ring through an oxygen or nitrogen atom in which said alkylene group may be substituted with amino, alkylamino or benzoyloxyamino, and said nitrogen atom may be substituted with acyl; and

Y is 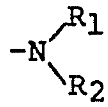 in which $R_1$ and $R_2$ are same or different, and represents hydrogen, lower alkyl, acyl, aminoalkyl or aminoacyloxy-alkyl; 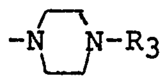 in which $R_3$ represents lower alkyl, cyclo alkyl, benzyl, carbamoyl lower alkyl, morpholinocarbonyl lower alkyl, pyrrolidinocarbonyl lower alkyl, piperidinocarbonyl lower alkyl, piperidino lower alkyl, anilinocarbonyl lower alkyl, alkylaminocarbonyl lower alkyl or alkoxycarbonyl lower alkyl; $-OR_4$ in which $R_4$ represents hydrogen, lower alkyl, aralkyl, succinimido, aminoacyloxyalkyl, aminoalkyloxyalkyl or aminoalkylcarbamoylalkyl; -NH-alkylene- 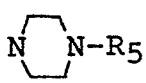 or -O-alkylene-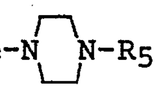 in which $R_5$ represents lower alkyl, morpholine lower alkyl, morpholinocarbonyl lower alkyl, pyrrodinocarbonyl lower alkyl, piperidinocarbonyl lower alkyl or lower alkylaminocarbonyl lower alkyl; or 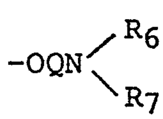 in which Q represents straight-chain or branched-chain alkylene, $R_6$ and $R_7$ each represent lower alkyl, or both jointly form, together with an adjacent nitrogen atom, pyrrolidino, piperidino or morpholino.

3. The medicine according to claim 1, or claim 2, characterised in that said synthetic phenyl ester type chymotrypsin inhibitor is present in an amount of 0.1 to 10 mg based on one unit of said insulin.

4. The medicine according to any of claims 1 to 3, characterised in that it is in the form of a preparation for oral or rectal administration.

SEPARATE CLAIMS FOR AUSTRIA

1. A method of preparing a medicine for the treatment of diabetes, characterised by the step of admixing insulin and a synthetic phenyl ester type chymotrypsin inhibitor.

2. The method according to claim 1, characterised in that said synthetic phenyl ester type chymotrypsin inhibitor is a compound represented by the following formula or an acid addition salt thereof,

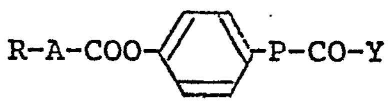

wherein R: indole which may be substituted with lower alkyl and/or lower alkoxy; naphthyl which may be substituted with lower alkoxy and which may be partially saturated with 2 or 4 hydrogen atoms; phenyl which may be substituted with lower alkyl, alkoxy, alkoxycarbonyloxy, acyl, acyloxy or halogen; cyclohexyl; or indane or furyl;

A: a single bond, alkylene or vinylene;

P: a single bond, alkylene, vinylene or alkylene attached to the benzene ring through an oxygen or nitrogen atom in which said alkylene group may be substituted with amino, alkylamino or benzoyloxyamino, and said nitrogen atom may be substituted with acyl; and

Y is 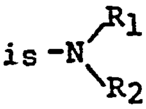 in which $R_1$ and $R_2$ are same or different, and represents hydrogen, lower alkyl, acyl, aminoalkyl or aminoacyloxyalkyl; 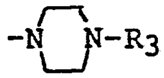 in which $R_3$ represents lower alkyl, cyclo alkyl, benzyl, carbamoyl lower alkyl, morpholinocarbonyl lower alkyl, pyrrolidinocarbonyl lower alkyl, piperidinocarbonyl lower alkyl, piperidino lower alkyl, anilinocarbonyl lower alkyl, alkylaminocarbonyl lower alkyl or alkoxycarbonyl lower alkyl; $-OR_4$ in which $R_4$ represents hydrogen, lower alkyl, aralkyl, succinimido, aminoacyloxyalkyl, aminoalkyloxyalkyl or aminoalkylcarbamoylalkyl; -NH-alkylene-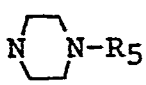 or -O-alkylene-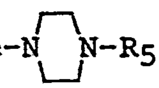 in which $R_5$ represents lower alkyl, morpholine lower alkyl, morpholinocarbonyl lower alkyl, pyrrodinocarbonyl lower alkyl, piperidinocarbonyl lower alkyl or lower alkylaminocarbonyl lower alkyl; or 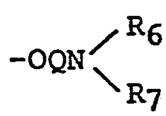 in which Q represents straight-chain or branched-chain alkylene, $R_6$ and $R_7$ each represent lower alkyl, or both jointly form, together with an adjacent nitrogen atom, pyrrolidino, piperidino or morpholino.

3. The method according to claim 1 or claim 2, characterised in that said synthetic phenyl ester type chymotrypsin inhibitor is present in an amount of 0.1 to 10 mg based on one unit of said insulin.

# FIG.1

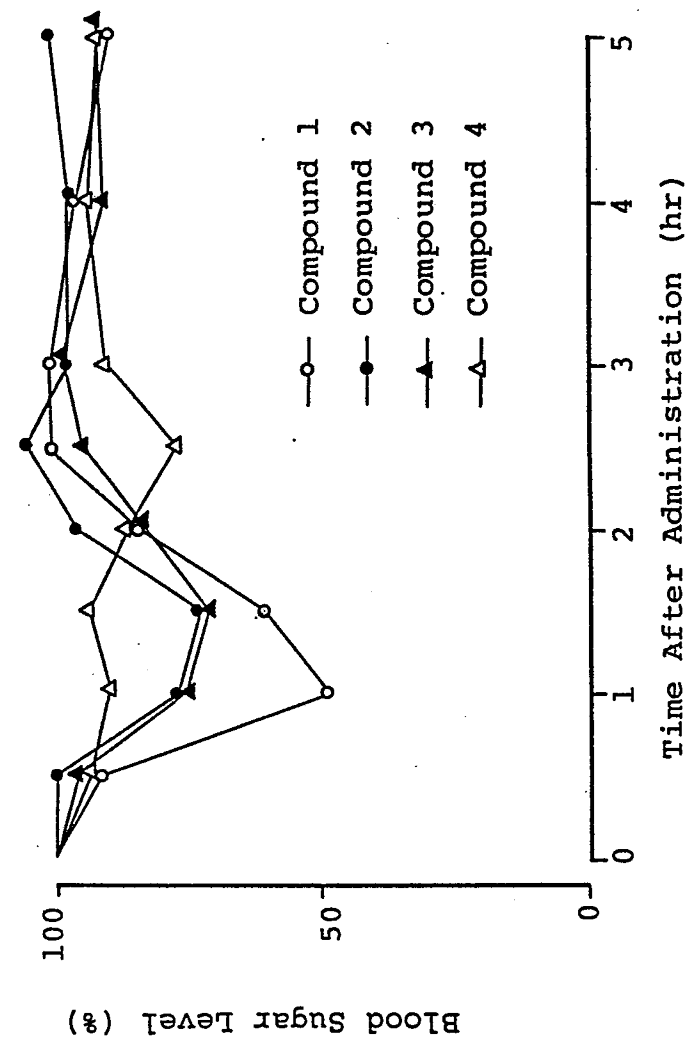
Compound 1
Compound 2
Compound 3
Compound 4
Blood Sugar Level (%)
100
50
0
Time After Administration (hr)
0
1
2
3
4
5

2/5

FIG. 2

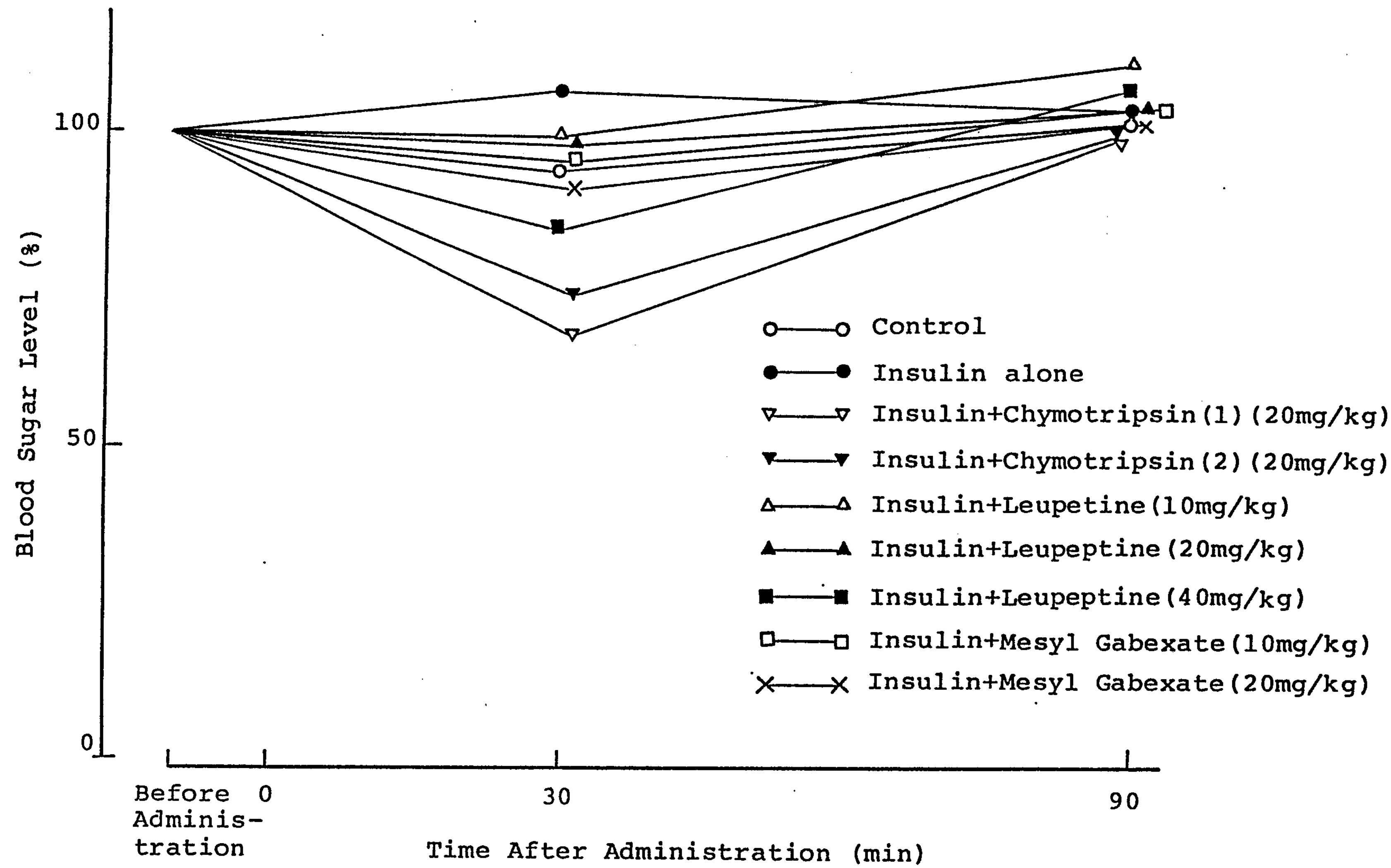
100
50
0
Blood Sugar Level (%)
Before Adminis-tration
0
30
90
Time After Administration (min)
Control
Insulin alone
Insulin+Chymotripsin(1)(20mg/kg)
Insulin+Chymotripsin(2)(20mg/kg)
Insulin+Leupetine(10mg/kg)
Insulin+Leupeptine(20mg/kg)
Insulin+Leupeptine(40mg/kg)
Insulin+Mesyl Gabexate(10mg/kg)
Insulin+Mesyl Gabexate(20mg/kg)

FIG. 3

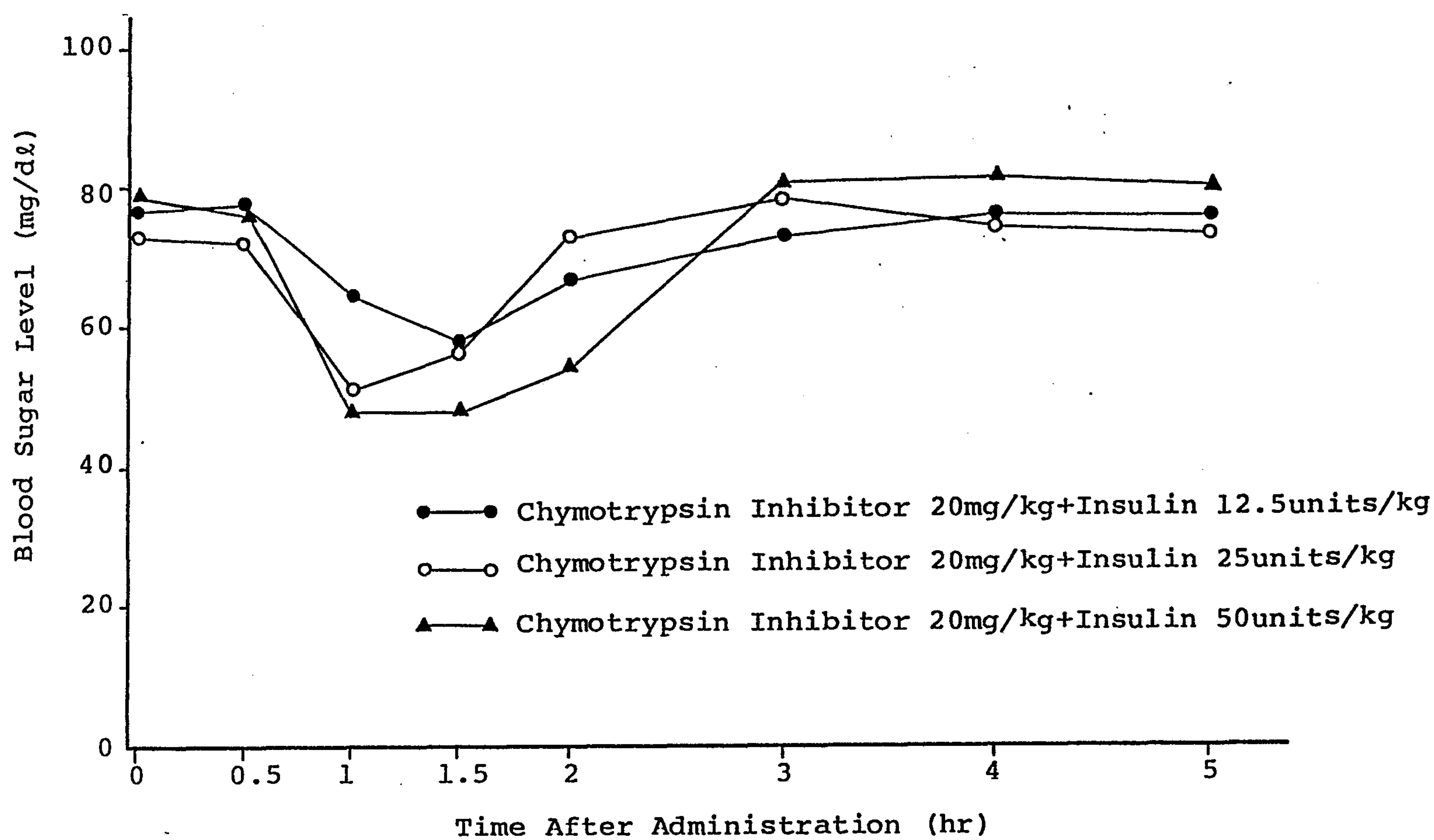
100
80
60
40
20
0
Blood Sugar Level (mg/dℓ)
0
0.5
1
1.5
2
3
4
5
Time After Administration (hr)
Chymotrypsin Inhibitor 20mg/kg+Insulin 12.5units/kg
Chymotrypsin Inhibitor 20mg/kg+Insulin 25units/kg
Chymotrypsin Inhibitor 20mg/kg+Insulin 50units/kg

4/5

# FIG. 4

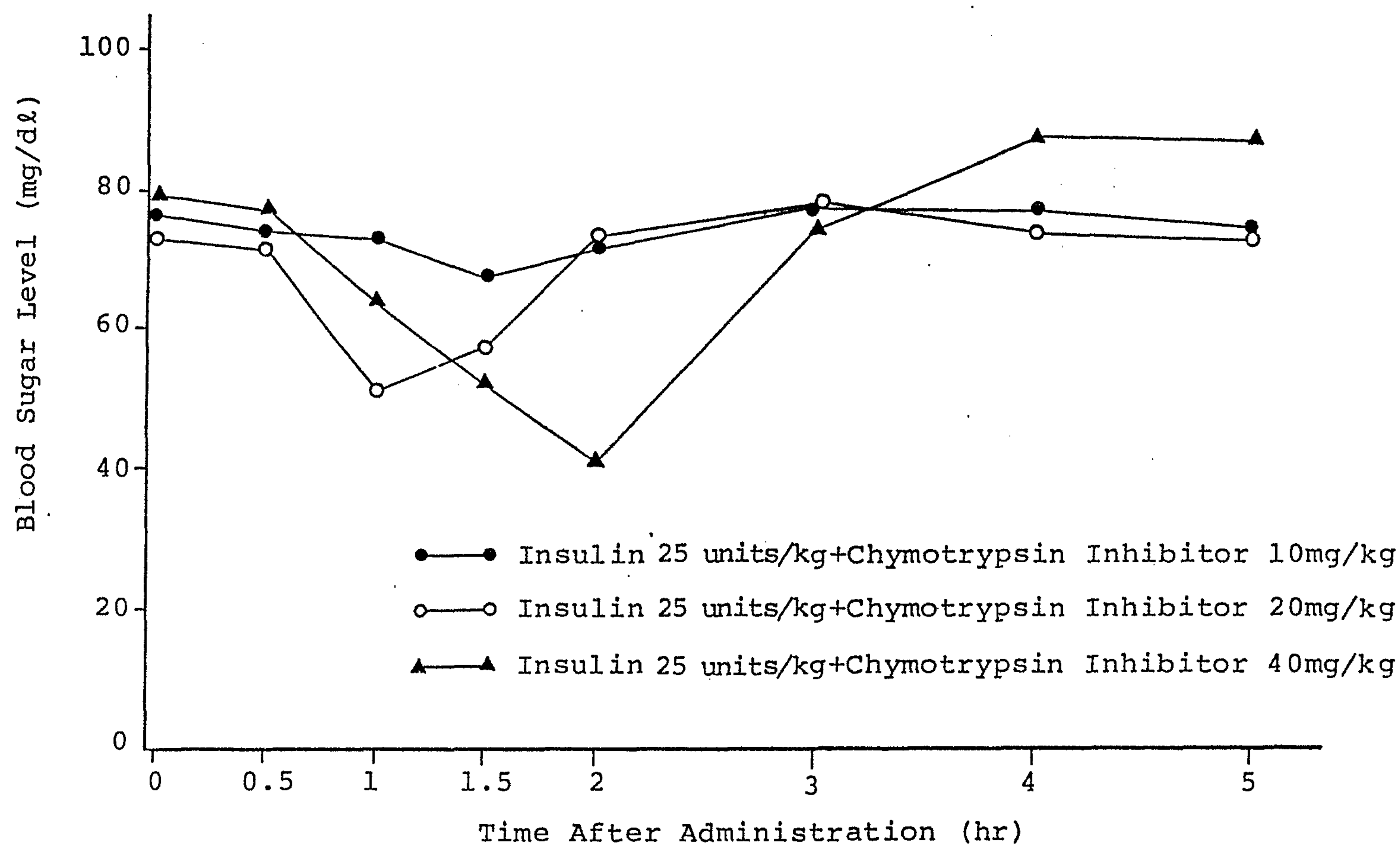
100
80
60
40
20
0
Blood Sugar Level (mg/dℓ)
Insulin 25 units/kg+Chymotrypsin Inhibitor 10mg/kg
Insulin 25 units/kg+Chymotrypsin Inhibitor 20mg/kg
Insulin 25 units/kg+Chymotrypsin Inhibitor 40mg/kg
0
0.5
1
1.5
2
3
4
5
Time After Administration (hr)

FIG. 5

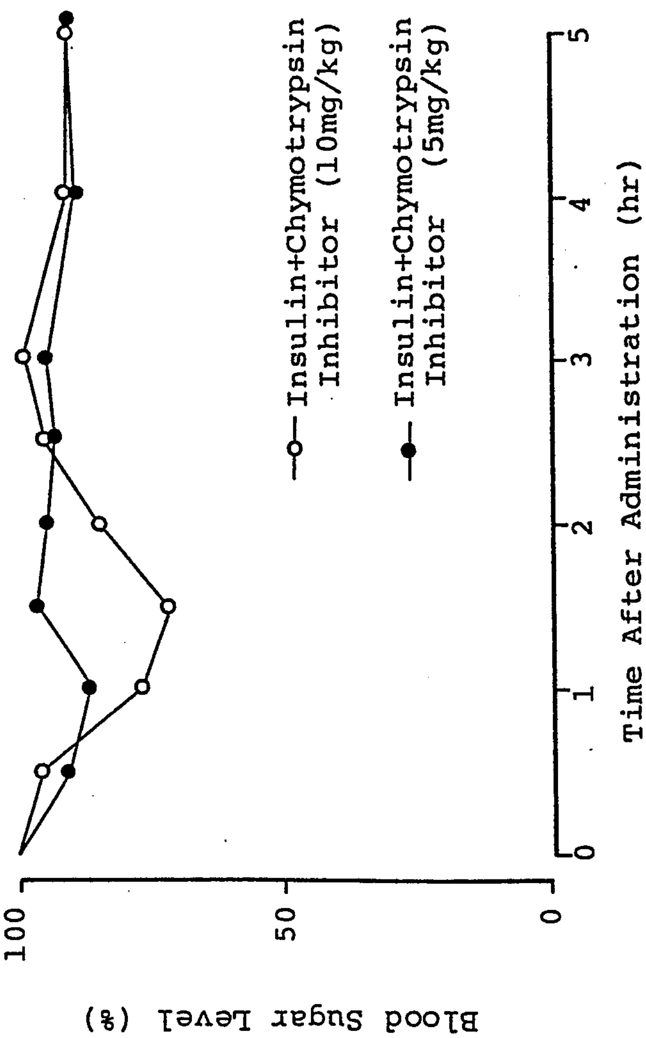
Blood Sugar Level (%)
100
50
0
0
1
2
3
4
5
Time After Administration (hr)
Insulin+Chymotrypsin Inhibitor (10mg/kg)
Insulin+Chymotrypsin Inhibitor (5mg/kg)

European Patent Office

# EUROPEAN SEARCH REPORT

0071433

Application number

EP 82 30 3902

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,A | CHEMICAL ABSTRACTS, vol. 89, no. 14, 2nd October 1978, page 540, column 1, no. 117816z, Columbus, Ohio, USA & JP - A - 78 15412 (ONO PHARMACEUTICAL CO., LTD.) 13-02-1978 * Abstract * | 1-3 | A 61 K 37/26<br>A 61 K 45/06<br>C 07 D 209/18<br>C 07 D 295/18 //<br>(A 61 K 37/26<br>A 61 K 31/535)<br>(A 61 K 37/26<br>A 61 K 31/495)<br>(A 61 K 37/26<br>A 61 K 31/405) |
| D,A | US-A-4 156 719 (HITOSHI SEZAKI) & JP - A - 78 107 408 | 1-3 | |
| D,A | PATENTS ABSTRACTS OF JAPAN, vol. 5, no. 24(C43)(696), 13th February 1981, page 23C43 & JP - A - 55 149 240 (KOUWA K.K.) 20-11-1980 | 1-3 | |
| | ----- | | TECHNICAL FIELDS SEARCHED (Int. Cl. 3) |
| | | | A 61 K<br>C 07 D |

The present search report has been drawn up for all claims

| Place of search | Date of completion of the search | Examiner |
|---|---|---|
| THE HAGUE | 22-10-1982 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82